# EUROPEAN PATENT SPECIFICATION

(11) **EP 2 696 881 B1**
(45) Date of publication and mention of the grant of the patent: **03.02.2016**
(21) Application number: 12713983.0
(22) Date of filing: 11.04.2012
(51) Int. Cl.: A61K 35/768, A61P 35/00

(54) **AVIAN METAPNEUMOVIRUS IN ONCOLYSIS**
AVIÄRES METAPNEUMOVIRUS IN DER ONKOLYSE
MÉTAPNEUMOVIRUS AVIAIRE DANS L'ONCOLYSE

(30) Priority: 12.04.2011 EP 11162008
(43) Date of publication of application: 19.02.2014
(73) Proprietor: Intervet International B.V., 5831 AN Boxmeer (NL)
(72) Inventor: SCHRIER, Carla, Christina, NL-5831 AN Boxmeer (NL)
(74) Representative: Intervet International B.V.
(86) International application number: PCT/EP2012/056506
(87) International publication number: WO 2012/140032

(56) References cited:
- US-A1- 2011 217 266
- VAHA-KOSKELA ET AL: "Oncolytic viruses in cancer therapy", CANCER LETTERS, NEW YORK, NY, US, vol. 254, no. 2, 8 September 2007 (2007-09-08), pages 178-216, XP022182282, ISSN: 0304-3835, DOI: 10.1016/J.CANLET.2007.02.002 cited in the application
- SABARA MARTA I ET AL: "Plaque assay for avian metapneumovirus using a Japanese quail fibrosarcoma cell line (QT-35).", JOURNAL OF VIROLOGICAL METHODS JAN 2003 LNKD- PUBMED:12445932, vol. 107, no. 1, January 2003 (2003-01), pages 9-14, XP002678577, ISSN: 0166-0934
- KONG B W ET AL: "Comparison of avian cell substrates for propagating subtype C avian metapneumovirus", VIRUS RESEARCH, AMSTERDAM, NL, vol. 116, no. 1-2, 1 March 2006 (2006-03-01), pages 58-68, XP024957024, ISSN: 0168-1702, DOI: 10.1016/J.VIRUSRES.2005.08.013 [retrieved on 2006-03-01]

## Description

The present invention relates to avian metapneumovirus (AMPV) for use in therapy and to pharmaceutical compositions comprising avian metapneumovirus (AMPV) for use in therapy.

It is known for several decades that some viruses may be able to eradicate tumors. Such viruses are commonly known as oncolytic viruses. Although the number of oncolytic tumors is relatively low, they are found in several viral genera. Oncolytic members of i.a. the following genera are known: adenovirus, herpesvirus, polyomavirus, poxvirus, Parvovirus, reovirus, Orthomyxovirus, paramyxovirus, rhabdovirus, coronavirus, picornavirus, togavirus and retrovirus. A recent mini-review by Vaha-Koskela, M. et al., gives an overview of oncolytic viruses in cancer therapy (Cancer Letters 254: 178-216 (2007)).

A problem faced with all oncolytic viruses is that after administration of a first dose, immunity against the virus used starts building up. Also, given the unpredictable differentiation and de-differentiation pathways in tumor cells, it may well be that some individual tumor cells of a certain tumor mass become resistant for an oncolytic virus used in a cancer therapy. This can e.g. be due to the fact that such tumor cells lose a receptor for that oncolytic virus.

If only for these reasons, there is a need for new oncolytic viruses, that can be used when starting a cancer therapy or as alternative when other oncolytic viruses fail to be effective in a cancer therapy.

It was surprisingly found now, that a live avian virus, Turkey Rhinotracheitis virus, presently also known as avian metapneumovirus (AMPV), unexpectedly has oncolytic effects on mammalian cells.

Turkey Rhinotracheitis (TRT) virus or avian metapneumovirus is a member of the metapneumovirus genus within the Paramyxoviridae virus family. Metapneumoviruses have a single-stranded non-segmented RNA genome of antisense polarity.
Within the Paramyxoviridae family, up till now, four genera of viruses were known to comprise an oncolytic member: one member within the genus Avulavirus (Newcastle Disease virus), one within the genus Morbillivirus (specific measles strains), one within the genus Respirovirus (Sendai virus) and one within the genus Rubulavirus (mumps virus).

The genus metapneumovirus of the Paramyxoviridae family was until now not known or suspected to have a member showing oncolytic properties.
The genus metapneumovirus has two members, one of which is AMPV mentioned above, the other member is human metapneumovirus (HMPV). Both viruses cause respiratory tract illness; HMPV in humans and AMPV in poultry.
It is the fusion protein (F) of metapneumoviruses that is responsible for the difference in tropism. It has been convincingly shown by De Graaf et al., that HMPV is not capable of infecting poultry. And *vice versa* AMPV has never been reported to cause disease or even any clinical symptoms in mammals. (De Graaf M., et al., J. Gen. Virol. 90:1408-1416 (2009))
Moreover, neither for HMPV nor for AMPV, any anti-tumor effect has been described.

Live AMPV selectively kills mammalian tumor cells but not mammalian normal differentiated cells and mammalian normal proliferating cells at the same dose. This characteristic to selectively kill mammalian tumor cells will further be referred to as an anti-tumor effect.

An anti-tumor effect means that individual cells of a tumor are killed by the virus, whatever the mode of action may be. Viruses can e.g. have an anti-tumor effect on cells because they are lytic to these cells: lytic virus strains are thought to damage the plasma membrane of infected cells. Another form of an anti-tumor effect is e.g. seen with non-lytic viruses; they appear to interfere with the metabolism of the cell and to cause the death of the cell due to this mode of action. The anti-tumor effect of AMPV makes the virus very suitable for use in cancer therapy.

Thus, a first embodiment of the present invention relates to a live avian metapneumovirus (AMPV) for use in cancer therapy in a mammal.

Preferably, AMPV is used in cancer therapy against breast, lung, prostate, glioblastoma, fibrosarcoma, ovarian, cervical, bladder or colon cancer or against melanoma.

More preferably, AMPV is used in cancer therapy against colon cancer.

In order to have an anti-tumor effect, the AMP virus has to be administered in an amount that is cytotoxic to tumor cells. This amount is referred to as a cytotoxic amount. Thus, the cytotoxic amount of AMPV is the amount of virus necessary for the induction of tumor cell death.

Theoretically spoken, one AMPV can infect and kill one tumor cell. Thus the cytotoxic amount of AMPV necessary for the induction of tumor cell death would be one virus per cell. In a practical setting, however, one would administer an amount that is a multitude of the number of tumor cells to be infected.

Nevertheless, very low amounts of virus would be needed to induce an anti-tumor effect. Numbers of 10³ plaque forming units (pfu) of virus per dose would already be sufficient to attack low amounts of tumor cells. Therefore, for many practical purposes, a number as low as 10³ pfu of virus could already be considered to be a cytotoxic amount.
However, it will be clear that if the amount of virus administered is so low that only a few tumor cells are infected, some time will pass before the first round of infection has led to new progeny virus that is capable of infecting further tumor cells. The same is true for subsequent replication rounds, and in the meantime an immune response against the virus will build up and interfere with the virus. Therefore, preferably higher numbers of virus would be administered at once. In that case, many tumor cells will become infected at the same time and thus many tumor cells will become infected before immunity against the virus builds up.
The very mild or even absent effects of the virus on non-tumor cells in mammals allows for such relatively high doses to be administered. So, although doses in the wide range from 10³ to 10¹² pfu would be acceptable doses, doses in the range from 10⁶ and 10¹² pfu would be preferred doses for most applications that would benefit from a high amount of virus. Doses in the range from 10⁹ to 10¹² pfu would be more preferred.

Another embodiment of the present invention relates to a pharmaceutical composition for use in cancer therapy in a mammal, characterised in that said pharmaceutical composition comprises a cytotoxic amount of live avian metapneumovirus (AMPV) and a pharmaceutically acceptable carrier.

The concept of a "pharmaceutically acceptable carrier" is explained elsewhere (*vide infra*).

Especially when a tumor is a massive tumor that has reached a thickness of several cell layers, the inner layers may not be directly exposed to viral attack. This is especially true for tumors with a low level of vascularization. Therefore, it is important that the progeny virus originating from killed tumor cells, or newly administered virus, is available for the infection of deeper cell layers within the tumor after the upper cell layers are killed.
For this reason, and in view of the fact that sooner or later an immune response will build up against AMPV, it might be beneficial to administer immunosuppressive agents to a patient, before and/or during the treatment with AMPV. This would postpone or suppress an immune response against the virus, so that subsequent rounds of infection can take place until all susceptible tumor cells are killed. Immunosuppressive agents are extensively known in the art. Examples of such immunosuppressive agents are i.a.: glucocorticoids inhibiting genes encoding interleukins and TNF-γ; cytostatics such as methotrexate and azathriopine; antibodies directed against CD25 and CD3 such as Dactinomycin; drugs acting on immunophilins such as ciclosporin and tacrolimus; and other drugs such as interferons, opioids, TNF binding proteins, mycophenolate and small biological agents such as Fingolimod and Myriocin. The use of such immunosuppressive agents would be indicated by the vendors of these agents.

Therefore, a preferred form of this embodiment relates to a pharmaceutical composition for use according to the invention, characterised in that said composition in addition comprises an immunosuppressive agent.

Immunosuppressive agents can be administered once, but they can also be administered in repeated doses over a longer period, e.g. in order to maintain the immunosuppressive effect over time.

Therefore, a pharmaceutical composition for use according to the invention, regardless if it comprises an immunosuppressive agent or not, is preferably administered to mammals that are subjected to a treatment with an immune suppressive agent.

As mentioned above, without the administration of immunosuppressive agents, sooner or later an immune response will build up against AMPV, which has negative consequences for the possibility to infect cells that were not infected by the virus in a first round of infection. This problem can be avoided in an alternative way through the administration of another (now non-AMP) virus having an anti-tumor effect (for examples, vide supra). Such viruses would not be blocked by an immune response against AMPV.

Preferably, the interval of time between the administration of AMPV and a non-AMPV (or a non-AMPV and AMPV, *vide infra*) will be between 2 and 56 weeks. The period of 2-56 weeks between the administration of the first and second virus has the following rationale: some tumors are fast growing, whereas other tumors, or even metastasized tumor cells can be slowly growing or even be "dormant" for quite some time. Thus, depending on the characteristics of the tumor, it could be beneficial to give a second virus earlier or later in time. In many cases, the period between the administration of the first and second virus would be shorter, because the time of "dormancy" is less than 56 week. And moreover, one might want to avoid an risks of earlier outgrowth of cells. Thus, a preferred period would be between 2 and 28 weeks, more preferred between 2-20, 2-16, 2-12 or even 2-8 weeks in that order of preference.

Thus, another form of this embodiment relates to a pharmaceutical composition for use in cancer therapy in a mammal according to the invention, characterised in that said cancer therapy comprises the step of administering a cytotoxic amount of live AMPV to said mammal, followed by the step of administering a cytotoxic amount of a non-AMPV to said mammal within 2-56 weeks of said administration of a cytotoxic amount of live AMPV.

It is clear that this use in cancer therapy can *mutatis mutandis* also be applied to animals that have been treated earlier with a non-AMPV. Such a use would apply to a therapy that comprises the step of administering a cytotoxic amount of live AMPV within 2-56 weeks after the administration of a cytotoxic amount of a non-AMPV.
Thus, again another form of this embodiment relates to a pharmaceutical composition for use in cancer therapy in a mammal according to the invention, characterised in that said cancer therapy comprises the step of administering a cytotoxic amount of live AMPV to said mammal within 2-56 weeks after the step of administering a cytotoxic amount of a non-AMPV to said mammal.

Of the oncolytic non-AMP viruses mentioned above, the virus most frequently used in cancer therapy is another cytotoxic paramyxovirus, Newcastle disease virus (NDV). Ample guidance for the use of NDV is given below.
Thus in a preferred embodiment, the non-AMPV is NDV.

Next to the use of immunosuppressive agents and the use of other cytotoxic (non-AMPV) viruses prior to or adjacent to the use of AMPV, there are several approached to enhance virus delivery to tumor cells. One approach is pre-treatment of tumor tissue with compounds such as proteolytic enzymes e.g. hyaluronidase and collagenase. (McKee, T.D. et al, Cancer Res. 66:2509-2513 (2006), Cairns, R. et al., Mol. Cancer Res. 4: 61-70 (2006), Minchinton, A.I. et al., Nat. Rev. Cancer 6: 583-592 (2006)).
Another approach to increase blood-tumor permeability is through administration of vaso-active or vaso-normalizing compounds such as bradikynin, paclitaxel or leukotrines.
The use of such compounds would be indicated by the vendors of the compounds.
Such treatment facilitates virus penetration and thus the delivery of virus to the tumor cells. Such compounds are further referred to as compounds that enhance virus delivery.

Therefore, another preferred form of this embodiment relates to a pharmaceutical composition for use according to the invention, characterised in that said composition in addition comprises a compound that enhance virus delivery.

Also, methods relying on physical methods that enhance virus delivery, e.g. by increasing the oxygenation of tumors have been proposed as a way of increasing the blood-tumor permeability. Such methods e.g. rely upon inhalation of hyperoxic gas or local hyperthermia. Such methods are further referred to as methods that enhance virus delivery.

Compounds that enhance virus delivery can be administered once, but they can also be administered in repeated doses over a longer period, e.g. in order to maintain the effect over time.

Therefore, a pharmaceutical composition for use according to the invention, regardless if it comprises a compound that enhances virus delivery or not, is preferably administered to mammals that are subjected to a treatment with a compound that enhances virus delivery.

Equally, methods that enhance virus delivery can be applied to an animal for a prolonged period of time.

Thus, a pharmaceutical composition for use according to the invention, regardless if it comprises a compound that enhances virus delivery or not, is preferably administered to mammals that are subjected to a treatment with a method that enhances virus delivery.

Another approach that can be successfully applied in combination with AMPV treatment according to the invention is the more classical approach to use cytostatic compounds. Such compounds are well-known in the art and they comprise alkylating agents such as chlorambucil and ifosfamide, antimetabolites such as mercaptopurine, plant alkaloids and terpenoids such as vincristine, podophyllotoxin and tannanes, and topoisomerase inhibitors such as irinotecan and amsacrine. And as is true for the immunosuppressive agents, enzymes and compounds mentioned above, the use of such enzymes or compounds would be indicated by the vendors of the cytostatic compounds.

Therefore, again another preferred form of this embodiment relates to a pharmaceutical composition for use according to the invention, characterised in that said composition in addition comprises a cytostatic compound.

Cytostatic agents can be administered once, but they can also be administered in repeated doses over a longer period, e.g. in order to maintain the cytostatic effect over time.
Therefore, a pharmaceutical composition for use according to the invention, regardless if it comprises a cytostatic agent or not, is preferably administered to mammals that are subjected to a treatment with a cytostatic compound.

With regard to the nature of the mammal the following can be said: it goes without saying that the use of the present invention for cancer therapy is very suitable for humans. The use in non-human mammals, i.e. for veterinary application would i.a. for economical reasons especially be applicable in companion animals such as equine, canine or feline species.

Therefore, another preferred embodiment relates to a pharmaceutical composition for use according to the invention, characterised in that the mammal belongs to a human, equine, canine or feline species.

With regard to the route or site of administration, in principle, the virus can be administered orally, through inhalation and by systemic application. Systemic application includes intramuscular , intraperitoneal, subcutaneous, intravenous and intra- or peri-tumoral administration.
For tumors in the respiratory tract, the intravenous route and the inhalation route would be the preferred routes.
For most other tumors, intravenous and/or intra-and/or peri-tumoral administration would be the preferred method of choice.
Therefore, another preferred form of this embodiment relates to a pharmaceutical composition for use according to the invention, characterised in that the site of administration of said pharmaceutical composition is intratumoral. Intratumoral administration is administration into the tumor mass.

Again another preferred form of this embodiment relates to a pharmaceutical composition for use according to the invention, characterised in that the site of administration of said pharmaceutical composition is peri-tumoral. Peri-tumoral administration is administration around the tumor mass.

Also, another preferred form of this embodiment relates to a pharmaceutical composition for use according to the invention, characterised in that the site of administration of said pharmaceutical composition is intravenous.

Still another preferred form of this embodiment relates to a pharmaceutical composition for use according to the invention, characterised in that the route of administration of said pharmaceutical composition is through inhalation.

The ample knowledge existing in the art with regard to the administration of another cytotoxic paramyxovirus, Newcastle disease virus (NDV) should also give the skilled person ample guidance. Merely as examples of the art, the following overview is provided:
In animal studies, NDV infection has been accomplished by i.a. intratumoral, intraperitoneal and intravenous route as reviewed in Schirrmacher V., Griesbach A., Ahlert T., Int. J. Oncol. 18: 945-52, 2001. NDV infection through the intramuscular or subcutaneous route has been reviewed by i.a.Heicappell R., Schirrmacher V., von Hoegen P., et al., Int. J. Cancer 37: 569-577 (1986). In human studies, in cases where patients have been infected with a lytic strain of NDV, intratumoral, intravenous or intramuscular injection has been used (Cassel W.A., Garrett R.E., Cancer 18: 863-868 (1965), Csatary L.K., Moss R.W., Beuth J., et al. Anticancer Res. 19: 635-638 (1999), Pecora A.L., Rizvi N., Cohen G.I., et al., J. Clin. Oncol. 20: 2251-2266 (2002), Csatary L.K., Bakács T., JAMA 281: 1588-1589 (1999), Wheelock E.F., Dingle J.H., N. Engl. J. Med. 271: 645-651 (1964), Csatary L.K., Lancet 2 (7728): 825 (1971). Also used are the following routes: inhalation and direct injection into the colon (i.e., via a colostomy opening). (Csatary L.K., Moss R.W., Beuth J., et al. Anticancer Res. 19: 635-638 (1999), Csatary L.K., Eckhardt S., Bukosza I., et al., Cancer Detect. Prev. 17: 619-27 (1993)).

The pharmaceutical composition for use according to the invention should in principle comprise the AMPV in a pharmaceutically acceptable carrier, in order to allow for the administration of the AMPV.

A "pharmaceutically acceptable carrier" is intended to aid in the effective administration of a compound, without causing (severe) adverse effects to the health of the animal to which it is administered. A pharmaceutically acceptable carrier can for instance be sterile water or a sterile physiological salt solution. In a more complex form the carrier can e.g. be a buffer, which can comprise further additives, such as stabilisers or conservatives. The nature of the carrier depends i.a. upon the route of administration. If the administration route is through inhalation, the carrier could be as simple as sterile water, a physiological salt solution or a buffer. If injection is the preferred route, the carrier should preferably be isotonic and have pH restrictions that make it suitable for injection. Such carriers however are extensively known in the art.

Details and examples are for instance described in well-known handbooks e.g.: such as: "Remington: the science and practice of pharmacy" (2000, Lippincot, USA, ISBN: 683306472). Examples of pharmaceutically acceptable carriers useful in the present invention include stabilizers such as SPGA, carbohydrates (e.g. sorbitol, mannitol, starch, sucrose, glucose, dextrin), proteins such as albumin or casein, protein containing agents such as bovine serum or skimmed milk and buffers (e.g. phosphate buffer). Preferably the stabiliser is free of compounds of animal origin, or even chemically defined, as disclosed in WO 2006/094,974. Especially when such stabilizers are added to the pharmaceutical composition, the pharmaceutical composition is very suitable for freeze-drying. Freeze-drying is a very suitable method to prevent AMPV from inactivation. Therefore, in a more preferred form, pharmaceutical compositions according to the invention are in a freeze-dried form.

### Legend to the figures:

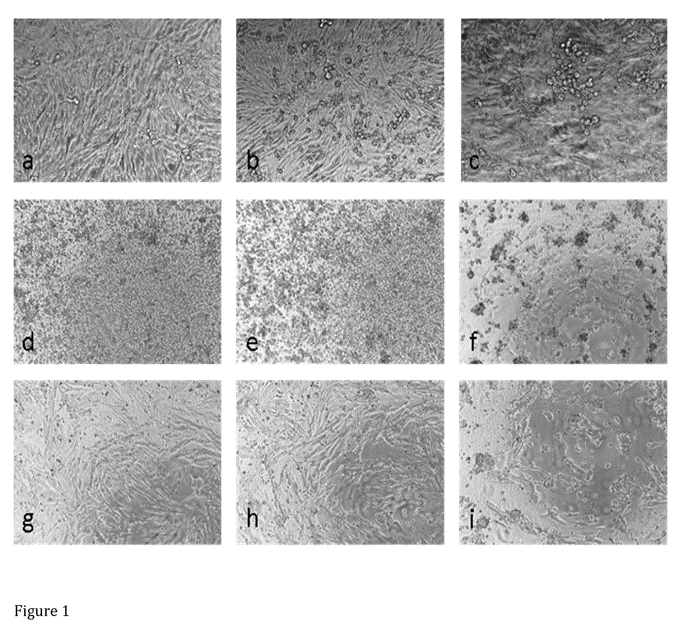

**Figure 1****: Manifestations of cell lysis after infection with TRT.**
Representative images of cell populations.
Picture a-c: CIPp cells, picture taken at 7 days post-infection.
Picture d-f: HMPOS cells, picture taken at 3 days post-infection.
Picture g-i: Mel-T4 cells, picture taken at 3 days post-infection.
Cells in a, d, g were mock-infected, cells in b, e, h were infected with a multiplicity of infection (MOI) of 0.1, cells in c, f, I were infected with an MOI of 1.

### Examples

### Example 1.

### Cell culture:

Human colon cancer cell line LS 174T with ATCC ordering number CL188 were grown according to the instructions supplied by the ATCC until semi-confluency.

### Pretreatment of virus:

Infections were performed with TRT virus suspensions in cell culture medium that were pretreated with trypsin as follows: 10 USP TU/ml trypsin was added to the virus suspensions and the mixture was incubated for 30 minutes. To inhibit trypsin activity 10% FBS (Biochrome AG) was added to the virus suspension.

### Infection of human colon cancer cell line LS 174T:

Culture medium was removed from the CL188 cells and 1 ml virus suspensions were added at multiplicity of infection (MOI) 0.1 and MOI 0.01. After 1 hour incubation at tissue culture conditions (37°C, 5% CO₂), 4 ml complete tissue culture medium, comprising 10% foetal calf serum, standard amounts of neomycin, pymafusin and tylosin as generally used in cell culture and 2ug/ml Fungizone (Gibco) was added and cells were kept at tissue culture conditions for 3 days. Then, the cell supernatant was harvested and stored at -70°C. Fresh complete tissue culture medium was added to the cells and at 7 days post-infection, cell supernatants were harvested. Subsequently, 1 ml PBS-red was added to the cells, which were then scraped to detach them from the cell culture surface. Finally, the virus titers (Log10 TCID50/ml) of the inoculate, both cell supernatant harvests and harvested cells were determined.

### Results

Table 1 shows the TRT titers (Log10 TCID50/ml) of the inoculate, the cell supernatants at 3 and 7 days post-inoculation and the harvested cells.

**Table 1: TRT virus titers.**

| *Log10 TCID50*/*ml* | **MOI 0.1** | **MOI 0.01** | **Neg. contr.** |
|---|---|---|---|
| inoculate | 5.2 | 3.4 | |
| supernatant 3dpi | 3.3 | 2.3 | |
| supernatant 7dpi | 3.1 | 1.8 | 0.0 |
| cells 7dpi | 4.6 | 3.5 | 0.0 |

The virus titers were then used to determine if virus replication had taken place during the course of infection. For that purpose, the absolute amounts of virus present in the inoculate, the cell supernatants and the cell harvest, were calculated. For the cell supernatants, this amount was corrected for the volume of the supernatants (5ml). The sum of the viral amounts in the cell supernatants at 3 and 7 days post-infection was added up to the amount of virus in the cells at 7 days post-infection. This total viral amount was divided by the amount of virus in the inoculate. A replication factor of >1 indicates viral amplification has taken place (Table 2).

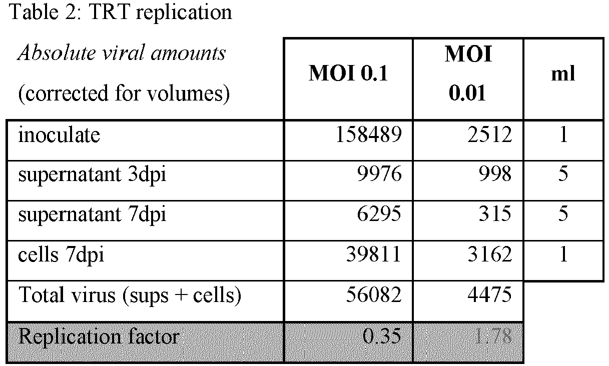

### Conclusion

Infection of CL188 human colon cancer cells with TRT at an MOI of 0.01 results in viral replication. Infection at an MOI of 0.1 did not allow the virus to replicate.

### Example 2.

To investigate the cytolytic effect of turkey rhinotracheitis virus (TRT) on canine tumour cells, three cell-lines were infected with TRT at two multiplicities of infection (MOIs). Uninfected cells served as a negative control. The occurrence of cell lysis was monitored and scored by microscopy from 3 days post-infection for 5 consecutive days.

### Materials

### Cell-lines:

- CIPp:: Canine mammary carcinoma cells, derived from a primary lesion, maintained in DMEM/F12, supplemented with 10% foetal bovine serum (FBS), Sodium Pyruvate and L-glutamine. Origin: Prof. Nobuo Sasaki, Laboratory of Veterinary Surgery, Graduate School of Agricultural and Life Sciences, University of Tokyo, Japan.
- HMPOS:: Highly metastatic canine osteosarcoma cells, maintained in RPMI1640, supplemented with 10% FBS and Sodium Pyruvate. Origin: Prof. dr. Jolle Kirpensteijn, Faculty of Veterinary Medicine, University of Utrecht, The Netherlands
- Mel-T4:: Canine melanoma cells, maintained in M199/F10, supplemented with 10% FBS and Sodium Pyruvate. Origin: MSD Animal Health, Boxmeer, The Netherlands.
- Virus:: Turkey rhinotracheitis virus (TRT), strain 1194 5.86 ¹⁰log TCID₅₀/ vial

### Methods

Cells were seeded in 96-wells tissue culture plates at 6000 cells per well (CIPp) or 15000 cells per well (HMPOS, Mel-T4). Cells were allowed to attach to the tissue culture plate and subsequently infected at MOI 1 or MOI 0.1 with TRT, diluted in PBS. Uninfected cells served as a negative control. After 30 minutes, medium (supplemented with 4% FCS) was added to all wells, resulting in a final concentration of FCS of 2%. The cells were incubated at 37°C, 5% CO₂. From 3 days post-infection, the cells were visually inspected for 5 consecutive days for the occurrence of cell lysis, using an Olympus CKX41 inverted phase-contrast microscope.

### Results

Cell lysis could be observed in all infected cell lines, albeit at various manifestations and degrees. In the infected CIPp populations, rounded cells were observed. Infected HMPOS cells grew in clusters, leaving gaps in the monolayer. Individual cells were rounded. Preferential growth in clusters and extensive disruption of the monolayer was also observed in infected Mel-T4 populations (Figure 1).

The level of cell lysis and the timing of its onset were observed to be dependent on both the cell-line and the MOI. The level of cell lysis increased over time. As is shown in Table 1, CIPp cells are most resistant to infection with TRT. At day 5 (MOI 1) or day 6 (MOI 0.1) the first signs of cell lysis became visible. The oncolytic effect of TRT on HMPOS cells is apparent already at 3 days post-infection at MOI 1. In contrast, infection at MOI 0.1 does not result in cell lysis. Mel-T4 cells are most sensitive to infection with TRT. Cell lysis was observed as soon as 3 days post-infection at MOI 1. For cells infected with MOI 0.1 this effect was delayed one day.

**Table 1: Gradual increase of cell lysis in canine tumour cells infected with TRT**

| | | | | | | | | | |
|---|---|---|---|---|---|---|---|---|---|
| | MOI 1 | MOI 0.1 | Medium | MOI 1 | MOI 0.1 | Medium | MOI 1 | MOI 0.1 | Medium |
| | - | - | - | + | - | - | + | - | - |
| | - | - | - | ++ | - | - | ++ | + | - |
| | + | - | - | ++ | - | - | +++ | + | - |
| | ++ | + | - | ++ | +/- | - | +++ | ++ | - |
| | ++ | + | - | +++ | - | - | +++ | ++ | - |

| | | | | | | | | | |
|---|---|---|---|---|---|---|---|---|---|
| - : no cell lysis, +/- : minor abnormalities (some round cells, a few cell clusters), + : mild cell lysis, ++ : substantial cell lysis, +++ : severe cell lysis | | | | | | | | | |

### Conclusion

TRT has a clear cytolytic effect on the canine tumour cell lines CIPp, HMPOS and Mel-T4. The level and timing of cell lysis are subject to the cell-line concerned and the administered MOI.

## Claims

1. Live avian metapneumovirus (AMPV) for use in cancer therapy in a mammal.

2. Pharmaceutical composition for use in cancer therapy in a mammal, **characterised in that** said composition comprises a cytotoxic amount of live avian metapneumovirus (AMPV) and a pharmaceutically acceptable carrier.

3. Pharmaceutical composition for use according to claim 2, **characterised in that** said composition in addition comprises an immunosuppressive agent.

4. Pharmaceutical composition for use according to claim 2 or 3, **characterised in that** said mammal is subjected to a treatment with immunosuppressing agents

5. Pharmaceutical composition for use according to claim 2, **characterised in that** said composition in addition comprises a cytostatic compound.

6. Pharmaceutical composition for use according to claim 2 or 5, **characterised in that** said mammal is subjected to a treatment with a cytostatic compound.

7. Pharmaceutical composition for use according to claim 2, **characterised in that** said composition in addition comprises a compound that enhances virus delivery.

8. Pharmaceutical composition for use according to claim 2 or 7, **characterised in that** said mammal is subjected to a treatment with a compound and/or a method that enhances virus delivery.

9. Pharmaceutical composition for use according to claim 2, **characterised in that** said cancer therapy comprises the step of administering a cytotoxic amount of live AMPV to said mammal, followed by the step of administering a cytotoxic amount of a live non-AMPV to said mammal within 2-56 weeks of said administration of a cytotoxic amount of AMPV.

10. Pharmaceutical composition for use according to claim 2, **characterised in that** said cancer therapy comprises the step of administering a cytotoxic amount of live AMPV to said mammal within 2-56 weeks after the step of administering to said mammal a cytotoxic amount of a non-AMPV.

11. Pharmaceutical composition for use according to any of claims 2-10, **characterised in that** the mammal belongs to a human, equine, canine or feline species.

12. Pharmaceutical composition for use according to any of claims 2-10, **characterised in that** the site of administration of said pharmaceutical composition is intratumoral.

13. Pharmaceutical composition for use according to any of claims 2-10, **characterised in that** the site of administration of said pharmaceutical composition is peri-tumoral.

14. Pharmaceutical composition for use according to any of claims 2-10, **characterised in that** the site of administration of said pharmaceutical composition is intravenous.

15. Pharmaceutical composition for use according to any of claims 2-10, **characterised in that** the route of administration of said pharmaceutical composition is through inhalation.

## Patentansprüche

1. Lebendes aviäres Metapneumovirus (AMPV) zur Verwendung in der Krebstherapie bei einem Säuger.

2. Pharmazeutische Zusammensetzung zur Verwendung in der Krebstherapie bei einem Säuger, **dadurch gekennzeichnet, dass** die Zusammensetzung eine zytotoxische Menge an lebendem aviärem Metapneumovirus (AMPV) und einen pharmazeutisch unbedenklichen Träger umfasst.

3. Pharmazeutische Zusammensetzung zur Verwendung nach Anspruch 2, **dadurch gekennzeichnet, dass** die Zusammensetzung zusätzlich ein Immunsuppressivum umfasst.

4. Pharmazeutische Zusammensetzung zur Verwendung nach Anspruch 2 oder 3, **dadurch gekennzeichnet, dass** der Säuger mit Immunsuppressiva behandelt wird.

5. Pharmazeutische Zusammensetzung zur Verwendung nach Anspruch 2, **dadurch gekennzeichnet, dass** die Zusammensetzung zusätzlich ein Zytostatikum umfasst.

6. Pharmazeutische Zusammensetzung zur Verwendung nach Anspruch 2 oder 5, **dadurch gekennzeichnet, dass** der Säuger mit einem Zytostatikum behandelt wird.

7. Pharmazeutische Zusammensetzung zur Verwendung nach Anspruch 2, **dadurch gekennzeichnet, dass** die Zusammensetzung zusätzlich eine Verbindung, die die Virusabgabe fördert, umfasst.

8. Pharmazeutische Zusammensetzung zur Verwendung nach Anspruch 2 oder 7, **dadurch gekennzeichnet, dass** der Säuger mit einer Verbindung und/oder einem Verfahren, die/das die Virusabgabe fördert, behandelt wird.

9. Pharmazeutische Zusammensetzung zur Verwendung nach Anspruch 2, **dadurch gekennzeichnet, dass** die Krebstherapie den Schritt des Verabreichens einer zytotoxischen Menge an lebendem AMPV an den Säuger, gefolgt von dem Schritt des Verabreichens einer zytotoxischen Menge eines lebenden Nicht-AMPV an den Säuger innerhalb von 2-56 Wochen der Verabreichung einer zytotoxischen Menge an AMPV umfasst.

10. Pharmazeutische Zusammensetzung zur Verwendung nach Anspruch 2, **dadurch gekennzeichnet, dass** die Krebstherapie den Schritt des Verabreichens einer zytotoxischen Menge an lebendem AMPV an den Säuger innerhalb von 2-56 Wochen nach dem Schritt des Verabreichens einer zytotoxischen Menge eines Nicht-AMPV an den Säuger umfasst.

11. Pharmazeutische Zusammensetzung zur Verwendung nach einem der Ansprüche 2-10, **dadurch gekennzeichnet, dass** der Säuger zu einer Menschen-, Pferde-, Hunde- oder Katzenart gehört.

12. Pharmazeutische Zusammensetzung zur Verwendung nach einem der Ansprüche 2-10, **dadurch gekennzeichnet, dass** die Verabreichungsstelle der pharmazeutischen Zusammensetzung intratumoral ist.

13. Pharmazeutische Zusammensetzung zur Verwendung nach einem der Ansprüche 2-10, **dadurch gekennzeichnet, dass** die Verabreichungsstelle der pharmazeutischen Zusammensetzung peritumoral ist.

14. Pharmazeutische Zusammensetzung zur Verwendung nach einem der Ansprüche 2-10, **dadurch gekennzeichnet, dass** die Verabreichungsstelle der pharmazeutischen Zusammensetzung intravenös ist.

15. Pharmazeutische Zusammensetzung zur Verwendung nach einem der Ansprüche 2-10, **dadurch gekennzeichnet, dass** der Verabreichungsweg der pharmazeutischen Zusammensetzung mittels Inhalation ist.

## Revendications

1. Métapneumovirus aviaire (AMPV) vivant destiné à être utilisé en thérapie du cancer chez un mammifère.

2. Composition pharmaceutique destinée à être utilisée en thérapie du cancer chez un mammifère, **caractérisée en ce que** ladite composition comprend une quantité cytotoxique de métapneumovirus aviaire (AMPV) vivant et un vecteur pharmaceutiquement acceptable.

3. Composition pharmaceutique destinée à être utilisée selon la revendication 2, **caractérisée en ce que** ladite composition comprend de plus un agent immunosuppresseur.

4. Composition pharmaceutique destinée à être utilisée selon la revendication 2 ou 3, **caractérisée en ce que** ledit mammifère est soumis à un traitement avec des agents immunosuppresseurs.

5. Composition pharmaceutique destinée à être utilisée selon la revendication 2, **caractérisée en ce que** ladite composition comprend de plus un composé cytostatique.

6. Composition pharmaceutique destinée à être utilisée selon la revendication 2 ou 5, **caractérisée en ce que** ledit mammifère est soumis à un traitement avec un composé cytostatique.

7. Composition pharmaceutique destinée à être utilisée selon la revendication 2, **caractérisée en ce que** ladite composition comprend de plus un composé qui améliore l'apport de virus.

8. Composition pharmaceutique destinée à être utilisée selon la revendication 2 ou 7, **caractérisée en ce que** ledit mammifère est soumis à un traitement avec un composé et/ou un procédé qui améliorent l'apport de virus.

9. Composition pharmaceutique destinée à être utilisée selon la revendication 2, **caractérisée en ce que** ladite thérapie du cancer comprend l'étape d'administration d'une quantité cytotoxique d'AMPV vivant audit mammifère, suivie de l'étape d'administration d'une quantité cytotoxique d'un non-AMPV vivant audit mammifère à moins de 2 à 56 semaines de ladite administration d'une quantité cytotoxique d'AMPV.

10. Composition pharmaceutique destinée à être utilisée selon la revendication 2, **caractérisée en ce que** ladite thérapie du cancer comprend l'étape d'administration d'une quantité cytotoxique d'AMPV vivant audit mammifère dans les 2 à 56 semaines après l'étape d'administration audit mammifère d'une quantité cytotoxique d'un non-AMPV.

11. Composition pharmaceutique destinée à être utilisée selon l'une quelconque des revendications 2 à 10, **caractérisée en ce que** le mammifère appartient à une espèce humaine, équine, canine ou féline.

12. Composition pharmaceutique destinée à être utilisée selon l'une quelconque des revendications 2 à 10, **caractérisée en ce que** le site d'administration de ladite composition pharmaceutique est intratumoral.

13. Composition pharmaceutique destinée à être utilisée selon l'une quelconque des revendications 2 à 10, **caractérisée en ce que** le site d'administration de ladite composition pharmaceutique est péri-tumoral.

14. Composition pharmaceutique destinée à être utilisée selon l'une quelconque des revendications 2 à 10, **caractérisée en ce que** le site d'administration de ladite composition pharmaceutique est intraveineux.

15. Composition pharmaceutique destinée à être utilisée selon l'une quelconque des revendications 2 à 10, **caractérisée en ce que** la voie d'administration de ladite composition pharmaceutique est par inhalation.
